# EUROPEAN PATENT SPECIFICATION

(11) **EP 1 599 438 B1**
(45) Date of publication and mention of the grant of the patent: **09.01.2008**
(21) Application number: 03815878.8
(22) Date of filing: 04.02.2003
(51) Int. Cl.: C07C 51/54, C07C 63/16, C07D 307/89, C07D 209/48

(54) **PREPARATION OF SUBSTITUTED PHTHALIC ANHYDRIDE, ESPECIALLY 4-CHLOROPHTALIC ANHYDRIDE**
ZUBEREITUNG VON SUBSTITUIERTEN PHTHALSÄUREANHYDRIDEN, IM BESONDEREN 4-CHLORPHTHALSÄUREANHYDRID
PREPARATION D'UN ANHYDRIDE PHTALIQUE SUBSTITUE ET PLUS PRECISEMENT D'UN ANHYDRIDE TETRACHLOROPHTALIQUE

(43) Date of publication of application: 30.11.2005
(73) Proprietor: GENERAL ELECTRIC COMPANY, Schenectady, NY 12345 (US)
(72) Inventor: ODLE, Roy, Ray, Mt. Vernon, IN 47620 (US); GUGGENHEIM, Thomas, Link, Mount Vernon, IN 47620 (US)
(74) Representative: Kihn, Pierre Emile Joseph
(86) International application number: PCT/US2003/003231
(87) International publication number: WO 2004/072012

(56) References cited:
- US-A- 5 003 088
- US-A- 5 229 482
- US-A- 5 322 954

## Description

### BACKGROUND OF THE INVENTION

This invention relates to a preparation of anhydrides. More particularly, it relates to a transimidation-based method for the preparation of anhydrides.

Polyetherimides are high heat engineering plastics having a variety of uses. As disclosed in U.S. Patent No. 5,229,482, one route for the synthesis of polyetherimides proceeds through a bis(4-chlorophthalimide) having the following structure (I) 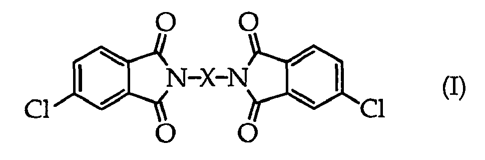 wherein X is a divalent, alkylene, cycloalkylene, or arylene moiety. The bis(4-chlorophthalimide) wherein X is a 1,3-phenyl group (II) is particularly useful.

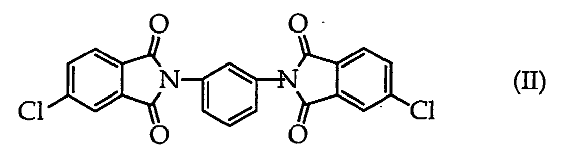

Bis(chlorophthalimide)s (I) and (II) are typically formed by the condensation of amines, e.g., 1,3-diaminobenzene with anhydrides, e.g., 4-chlorophthalic anhydride (III)

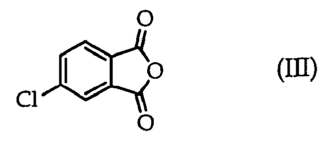

4-Chlorophthalic anhydride is an expensive starting material which presently must be custom synthesized. Current routes for the synthesis of 4-chlorophthalic anhydride lead to mixtures of isomers, which are difficult to separate, or are prohibitively costly. For example, the Diels-Alder condensation of maleic anhydride with 2-chloro-1,3-butadiene to yield 4-chlorotetrahydrophthalic anhydride, followed by aromatization in the presence bromine requires the subsequent recovery of HBr. Attempted thermal aromatization of 4-chloro-tetrahydrophthalic anhydride results in low yields of 4-chlorophthalic anhydride and tar formation. This and other routes are described in U.S. Patent Nos. 5,059,697; 5,003,088; 5,322,954; 4,978,760 and 5,233,054.

### BRIEF SUMMARY OF THE INVENTION

A new method for the synthesis of phthalic anhydrides (IV) 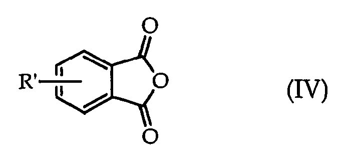 wherein R' is a halogen, an aromatic or aliphatic group comprising 1 to about 18 carbons, a hydrogen or a nitro group is the transimidation between the corresponding N-alkyl phthalimide (V) 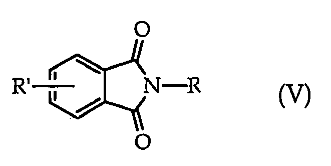 wherein R' in (IV) and R' in (V) are identical, and further wherein R is an alkyl group having from 1 to about 18 carbons, and a tetrahydrophthalic anhydride (VI)

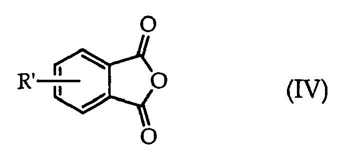

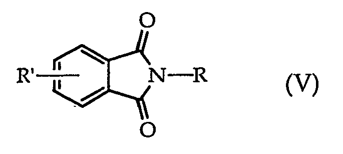

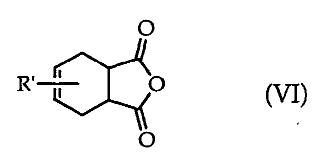

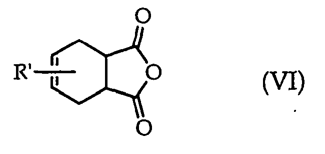

The by-product of the transimidation is an N-alkyl tetrahydrophthalimide (VII)

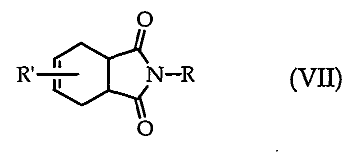

In an advantageous feature of this method, where R' in (V), (VI) and (VII) are identical, N-alkyl tetrahydrophthalimide (VII) may be converted by aromatization to produce the corresponding N-alkyl phthalimide (V).

### DETAILED DESCRIPTION OF THE PREFERRED EMBODIMENTS

A convenient route for the formation of phthalic anhydrides (IV) is via transimidation between the corresponding N-alkyl phthalimide (V) and a tetrahydrophthalic anhydride (VI). A by-product of this reaction is an N-alkyl tetrahydrophthalimide (VII). This by-product is preferably converted to yield the corresponding N-alkyl phthalimide (V).

The product phthalic anhydrides (IV) have the structure 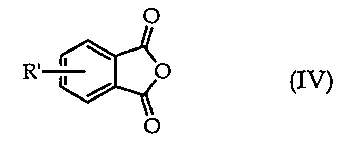 wherein R' is a halogen, an aromatic or aliphatic group comprising 1 to about 18 carbons, a hydrogen or a nitro group.

The starting N-alkyl phthalimides (V) have the structure 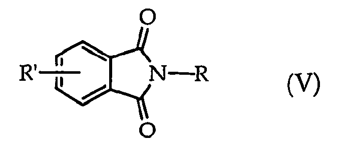 wherein R' is as described above, and R is a straight chain or branched alkyl group having from 1 to about 18 carbons, for example methyl, ethyl, propyl, and the like. N-alkyl phthalimides (V) may be obtained from the corresponding N-alkyl tetrahydrophthalimide by aromatization. Aromatization may be achieved by any method known in the art, such as those taught by U.S. Patent Nos. 5,233,054; 5,003,088; 5,059,697 and 4,978,760. Aromatization may also be achieved by passing the N-alkyl tetrahydrophthalimide over a transition metal catalyst such as V₂O₅ at a temperature in the range of about 250°C to about 270°C. Alternately, N-alkyl phthalimides (V) may be obtained by heating a tetrahydrophthalic anhydride with the desired alkyl amine or aryl amine at a temperature of about 50°C to about 250°C for up to about 5 hours. The resulting product, N-alkyl phthalimide may be isolated by any method known in the art such as distillation or column chromatography.

Tetrahydrophthalic anhydrides (VI) 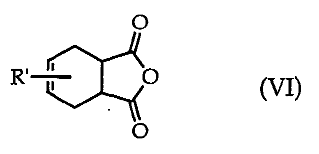 wherein R' is as defined above may be obtained by the Diels-Alder condensation of dienophile maleic anhydride with a diene substituted by R'. Conditions for this reaction are known in the chemical literature. Suitable R' substitutions include, but are not limited to, halogen, aromatic or aliphatic groups comprising 1 to about 18 carbons, hydrogen or nitro group. A preferred diene is 2-chloro-1,3- butadiene (chloroprene).

An advantageous feature of this method is that where R' are identical in both (V) and (VI), the by-product of the transimidation, N-alkyl tetrahydrophthalimide (VII) 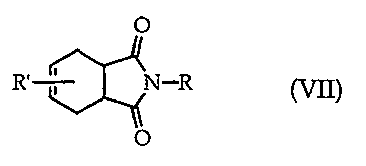 can be converted by aromatization to produce N-alkyl phthalimide (V) as discussed above. Due to this advantageous feature it is contemplated that the synthesis of 4-chlorophthalic anhydrides may be practiced in a batchwise or continuous fashion.

As shown in Scheme I this method is particularly suitable for the formation of 4-chlorophthalic anhydride (III), which is an important intermediate in the synthesis of polyetherimides.

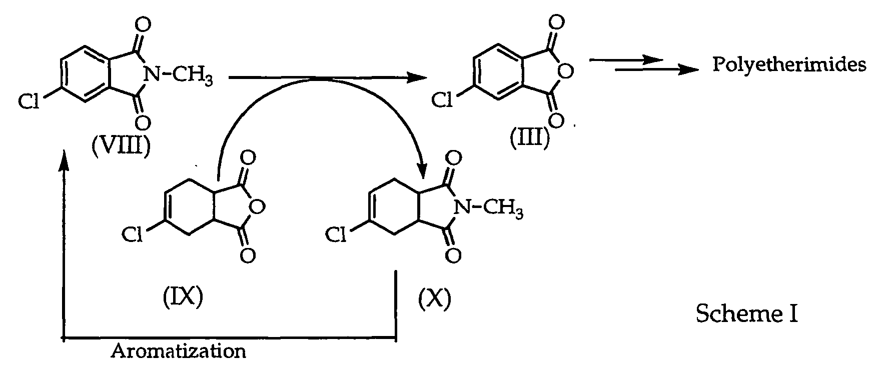

Accordingly, 4-chlorophthalic anhydride (III) is produced by transimidation of N-methyl-4-chlorophthalimide (VIII) with 4-chlorotetrahydrophthalic anhydride (IX). The by-product of the transimidation, N-methyl-4-chlorotetrahydrophthalimide (X) is preferably converted by aromatization to N-methyl-4-chlorophthalimide (VIII).

The invention is further described by the following non-limiting examples:

### EXAMPLES

### Example 1. Synthesis of N-methyl-4-chlorophthalimide (VIII).

An appropriately equipped glass reaction vessel was charged with 186.5 grams (1 mole) of 4-chlorotetrahydrophthalic anhydride and heated to 150°C under nitrogen. Methylamine gas (32 grams, 1.03 mole) was then introduced to the reaction vessel subsurface over 30 minutes. The reaction mixture was then heated for 3 hours at 180°C. The product was then distilled to give N-methyl-4-chlorophthalimide in approximately 95% yield.

### Example 2. Synthesis of 4-Chlorotetrahydrophthalic Anhydride (IX).

156.9 g (1.6 mole) of maleic anhydride and 250 ml toluene were added to a flask. 25 ml of toluene was removed by distillation to dry the solution. 150 g (1.69 mole) of chloroprene dissolved in 250 ml of xylene was slowly added to the flask. The entire addition took 30 minutes. The resulting solution was then heated at 55°C for three hours. The solution was then distilled to remove solvents. The remaining material was distilled at 160-165 °C (1 to 2 mm pressure) to afford 4-chlorotetrahydrophthalic anhydride in 85% yield (253 grams).

### Example 3. Synthesis of 4-Chlorophthalic Anhydride (III)

A resealable 50-ml stainless steel tube was charged with 1.5 g 4-chlorotetrahydrophthalic anhydride, 0.262 g N-methyl-4-chlorophthalimide, 0.841 g triethylamine, and 20 ml of water. The tube was sealed and heated in an oil bath at 170 °C for 3 hours, and then cooled to room temperature.

A small sample of the aqueous phase was analyzed by GCMS. The analysis showed that 83.2% exchange had occurred. Analysis further showed that the reaction mixture was composed of 2.4 mol% of N-methyl-4-chlorophthalimide, 11.9 mol% of N-methyl-4-chloro tetrahydrophthalimide, 11.9 mol% of 4-chlorophthalic anhydride (as the triethylamine salt of the corresponding diacid), and 73.8 mol% of 4-chlorotetrahydrophthalic anhydride (as the triethylamine salt of the corresponding diacid).

The aqueous phase was extracted with 20 ml of toluene containing 3 wt% triethylamine in a separatory funnel at room temperature. The toluene extraction effectively removed the N-methyl-4-chlorotetrahydrophthalimide and the unreacted N-methyl-4-chlorophthalimide from the aqueous phase. The aqueous phase still contained the 4-chlorophthalic anhydride (as the triethylamine salt of the corresponding diacid), and the 4-chlorotetrahydrophthalic anhydride (as the triethylamine salt of the corresponding diacid). The aqueous phase was distilled, during which the triethylamine salts cracked to liberate water and triethylamine, with the formation of 4-chlorophthalic anhydride and 4-chlorotetrahydrophthalic anhydride. The water and triethylamine were taken overhead, and the still bottoms were collected. The still bottoms were then distilled to separate the 4-chlorotetrahydrophthalic anhydride from the 4-chlorophthalic anhydride A. The 4-chlorotetrahydrophthalic anhydride was recombined with the water and TEA previously collected, and reused.

### Example 4. Conversion of N-methyl-4-chlorotetrahydrophthalimide (X) to N-methyl-4-chlorophthalimide.

Gas phase reactions were carried out in a hot-tube reactor that was packed with about 13 grams of a catalyst containing V₂O₅. The inlet of the hot-tube reactor was connected to a flow controller and heated syringe pump. The flow controller managed the flow of purified air. The heated syringe pump contained 4-chloro-N-methyl tetrahydrophthalimide and delivered it to the hot tube reactor at a constant rate of 0.05 milliliters per minute. The outlet of the hot tube reactor was connected to a receiver cooled in an ice-bath where the reaction products were collected. The hot-tube reactor was maintained at the 260°C. The reaction product was analyzed by gas chromatographic techniques after the system had equilibrated for 10-20 minutes. At a flow rate of 90ml/min all of the N-methyl-4-chlorotetrahydrophthalimide was converted to N-methyl-4-chlorophthalimide.

## Claims

1. A method for preparing a phthalic anhydride, comprising:
transimidation of an N-alkyl phthalimide (V) 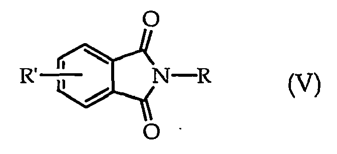
wherein R' is selected from the group consisting of halogens, aromatic groups comprising 6-18 carbons, aliphatic groups comprising 1-18 carbons, hydrogen and nitro groups, and R is an alkyl group having from 1 to 18 carbons, with substituted tetrahydrophthalic anhydride (VI) 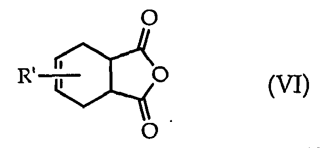
wherein R' is selected from the group consisting of halogen, aromatic groups comprising 6-18 carbons, or aliphatic group comprising 1-18 carbons, hydrogen and nitro groups, to yield a substituted N-alkyl tetrahydrophthalimide (VII) 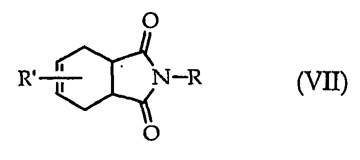
and a phthalic anhydride (IV)

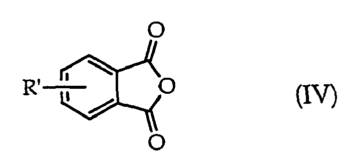

2. The method of claim 1, wherein the phthalic anhydride is 4-chlorophthalic anhydride and the N-alkyl phthalimide is a N-alkyl-4-chlorophthalimide.

3. The method of claim 1, further comprising converting the N-alkyl tetrahydrophthalimide by aromatization to the corresponding N-alkyl phthalimide.

4. The method of claim 3, wherein the N-alkyl tetrahydrophthalimide is converted to the corresponding N-alkyl phthalimide in the presence of vanadium oxide.

5. The method of claim 1, wherein the N-alkyl tetrahydrophthalimide (VII) is N-methyl-4-chlorotetrahydrophthalimide.

## Patentansprüche

1. Verfahren zur Herstellung von Phthalsäureanhydrid bei welchem man:
N-Alkylphthalimid (V) transimidiert 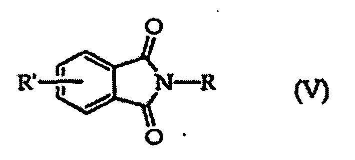
worin R' ausgewählt ist aus der Gruppe bestehend aus Halogen, aromatischen Gruppen aufweisend 6 - 18 Kohlenstoffe, aliphatischen Gruppen aufweisend 1 - 18 Kohlenstoffe, Wasserstoff und Nitrogruppen, und R eine Alkygruppe ist mit 1 bis 18 Kohlenstoffen,
mit substituiertem Tetrahydrophthalsäureanhydrid (VI) 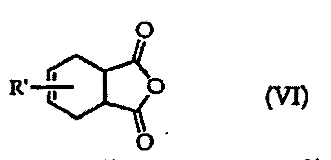
worin R' ausgewählt ist aus der Gruppe bestehend aus Halogen, aromatischen Gruppen umfassend 6 - 18 Kohlenstoffe, oder aliphatischen Gruppen umfassend 1 - 18 Kohlenstoffe, Wasserstoff und Nitrogruppen,
um ein substituiertes N-Alkyltetrahydrophtalimid (VII) zu ergeben 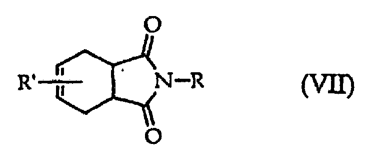
und ein Phthalsäureanhydrid (IV)

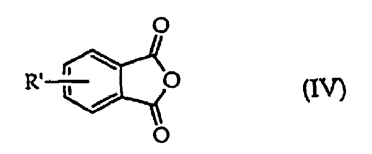

2. Verfahren nach Anspruch 1, wobei das Phthalsäureanhydrid 4-Chlorphthalsäureanhydrid ist und das N-Alkylphthalimid ein N-Alkyl-4-chlorphthalimid ist.

3. Verfahren nach Anspruch 1, weiterhin aufweisend, dass man das N-Alkyltetrahydrophthalimid durch Aromatisierung zum entsprechenden N-Alkylphtalimid konvertiert.

4. Verfahren nach Anspruch 3, wobei das N-Alkyltetrahydrophthalimid in Gegenwart von Vanadiumoxid zum entsprechenden N-Alkylphthalimid konvertiert wird.

5. Verfahren nach Anspruch 1, wobei das N-Alkyltetrahydrophthalimid (VII) N-Methyl-4-Chlortetrahydrophthalimid ist.

## Revendications

1. Procédé de préparation d'un anhydride phtalique qui comprend :
la transimidation d'un N-alkyl phtalimide (V) 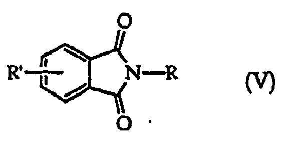
dans lequel R' est sélectionné dans l'ensemble comprenant les atomes d'halogène , les groupes aromatiques comprenant 6 - 18 atomes de carbone, les groupes aliphatiques comprenant 1 - 18 atomes de carbone, l'atome d'hydrogène et les groupes nitro, et R représente un groupe alkyle ayant 1 à 18 atomes de carbone,
avec un anhydride tétrahydrophtalique substitué (VI) 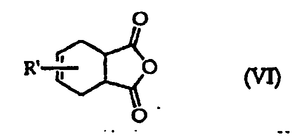 dans lequel R' est sélectionné dans l'ensemble comprenant les atomes d'halogène , les groupes aromatiques comprenant 6 - 18 atomes de carbone, les groupes aliphatiques comprenant 1 - 18 atomes de carbone, l'atome d'hydrogène et les groupes nitro, pour obtenir un N-alkyl tétrahydrophtalimide substitué (VII) 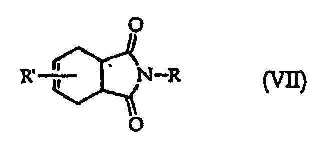 et un anhydride phtalique (IV)

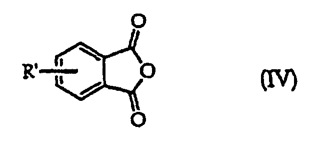

2. Procédé selon la revendication 1, dans lequel l'anhydride phtalique est l'anhydride 4-chlorophtalique et le N-alkyl phtalimide est un N-alkyl-4-chlorophtalimide.

3. Procédé selon la revendication 1, qui comprend en outre la conversion du N-alkyl tétrahydrophtalimide par aromatisation en le N-alkyl phtalimide correspondant.

4. Procédé selon la revendication 3, dans lequel le N-alkyl tétrahydrophtalimide est transformé en le N-alkyl phtalimide correspondant en présence d'oxyde de vanadium.

5. Procédé selon la revendication 1, dans lequel le N-alkyl tétrahydrophtalimide (VII) est le N-méthyl-4- chlorotétrahydrophtalamide.
